# EUROPEAN PATENT APPLICATION

(11) **EP 0 533 469 A1**
(43) Date of publication of application: **24.03.1993**
(21) Application number: 92308458.6
(22) Date of filing: 17.09.1992
(51) Int. Cl.: C12N 15/86, C12N 15/56

(54) **Expression vector and silkworm larvae transformant containing the same**

(30) Priority: 18.09.1991 US 761644
(71) Applicant: HONG KONG TECH COMPANY LIMITED, Hong Kong (HK)
(72) Inventor: Yukun, Sun, Shanghai (CN)
(74) Representative: Collier, Jeremy Austin Grey (GB)

(57) **Abstract**

An expression vector (and transformant harbouring such vector) has a foreign promoter and Bombyx mori DNA is useful to transform silkworm larvae. Also shown are processes for expressing a gene using the expression vector and for producing the expression vector.

## Description

### BACKGROUND OF THE INVENTION

### 1. Field of the Invention

The present invention relates to expression vectors and transformed hosts containing such expression vectors. More particularly, the present invention relates to virus expression vectors for use in silkworm larvae and transformed silkworm larvae containing those vectors, as well as to methods of producing polypeptides using such transformants and methods of producing those vectors.

### 2. Background of the Prior Art

The theories of genetic engineering and the expression of synthetic or natural polypeptides are now generally well-understood in the art. That is, it is commonly known that to produce polypeptides by recombinant engineering it is required to construct or isolate DNA which encodes the desired target polypeptide (a "structural gene"), to insert the structural gene into an expression vector under the control of a promoter system and to transform a suitable host cell to express the target polypeptide. Frequently, the expression vector contains a promoter/operon to place the structural gene under some sort of useful external control. Additionally, the expression vector often contains additional genetic information encoding some material which is essential for cell growth and/or reproduction. Thus, use of host cells which are deficient in producing that essential material ensures that the only cells which are grown are successfully infected, thereby optimizing the yield of transformants and ultimately, the target polypeptide.

Most frequently, common bacteria such as E. coli Brevibacterium and the like are utilized as host cells for expression. However, it is understood that polypeptides are glycosylated when produced natively in mammals such as humans. The glycosylation patterns play important roles in the function of many target polypeptides when such polypeptides are utilized as therapeutic agents in humans. For instance, deleting or varying the glycosylation pattern of a polypeptide may change its physical conformation and thus, will frequently affect its antibody-stimulating response upon administration. Accordingly, it would be desirable to utilize an expression host which expresses the natural glycosylated target polypeptides.

One deficiency of using bacteria to express target polypeptides in that they do not glycosylate such materials. To the contrary, yeast is a host which glycosylates polypeptides and is frequently used as an expression host. However, it is known that yeast does not secrete polypeptides due to the particular structural properties of its cell wall. Therefore, it is necessary to lyse the yeast cell wall and extract the expressed target polypeptide from the disrupted yeast cell. Unfortunately, this batchwise lysing and subsequent extraction interferes with continuous systematic production.

Other drawbacks of using yeast are that yeast cells tend to degrade materials which are not useful for the cells to thrive, and moreover, yeast cells often fail to properly assemble the large polypeptides. Such polypeptides include TPA and the like, and are those which are generally of therapeutic interest. Yeast is thought to degrade unnecessary polypeptides because they are not secreted, and additionally yeast is thought to improperly assemble large, bulky polypeptides because ensuring the proper synthesis of such materials often require chaperon proteins and highly developed endoplasmic reticulum systems. Moreover, the fact that it is can be difficult to maintain yeast culture in log phase growth virtually ensures that it may not be the preferred host cell of choice for practicing commercial scale biosynthesis.

Although mammalian cells overcome the problems involved with polypeptide production in yeast such as secretion and polypeptide biosynthesis, the cell lines frequently die out or become abnormal after a few generations. While this particular difficulty is overcome by the availability of human hybridoma lines, even these cells require notoriously stringent culture conditions. Moreover, the fact that the hybridomas originate in part from cancer cells together with current knowledge concerning retroviruses makes it clear that these cells can pose serious biohazard risks to both the researcher and in industry.

Accordingly, it is still considered desirable to obtain another host cell for expressing and excreting glycosylated target polypeptides which does not pose a health risk, does not require stringent culture conditions and maintains stable cell lines. To date, such has not been accomplished due to a lack of availability of a suitable expression vector.

### SUMMARY OF THE INVENTION

It is an object of the present invention to provide a vector for expressing polypeptides in silkworm larvae.

It is another object of the present invention to provide a silkworm larvae host which is transformed with an expression vector.

It is a still further object of this invention to provide a method of producing polypeptides using transformed silkworm larvae.

It is an additional object of the present invention to provide a method of producing vectors for expressing polypeptides in silkworm larvae transformants.

In accordance with an aspect of the present invention is an expression vector, comprising a foreign promoter and Bombyx mori NPV DNA.

In accordance with another aspect of the present invention is a transformed silkworm larvae harboring an expression vector, comprising a foreign promoter and Bombyx mori NPV DNA.

Similarly, a further aspect of the present invention is provided by a process for expressing a gene encoding a polypeptide, comprising the steps of: selecting an expression vector comprising a foreign promoter and Bombyx mori NPV DNA; transforming a silkworm larvae with said expression vector; culturing said silkworm larvae; and recovering said polypeptide.

An additional aspect of the invention is a process for producing an expression vector, comprising the steps of:
selecting DNA encoding a target polypeptide;
inserting said DNA into a pACNPV vector to create a shuttle vector;
combining said shuttle vector with BmNPV in solution to create a DNA mixture;
transfecting Bm-N cell with said DNA mixture; and isolating recombinant AcBmNPV virus from placques formed in said Bm-N cells.

The above and other objects of the present invention will become apparent when considering the detailed description of the preferred embodiments which follow.

### DETAILED DESCRIPTION OF THE PREFERRED EMBODIMENTS

Nucleopolyhedrosis virus (NPV) is a group of baculoviruses that infect different insect species but do not infect vertebrates or plants. Moreover, NPV isolated from one insect species generally is not infective in other insect species. This lack of cross-species infectivity has until now constrained efforts to extend the currently employed baculovirus AcNPV gene expression systems from Autographa californica to other insect species.

The NPV genome generally consists of a supercoiled double stranded circular DNA approximately 128 Kb in length. During NPV replication, the virus is packaged and shed from the cells or accumulates in polyhedron encased cellular inclusion bodies. Polyhedrin is encoded by a late viral gene of NPV and has a molecular weight about 30 kDa. Polyhedrin accumulates in the infected cells at high levels (1 mg/ml per 1-2 x 10⁶ cells), about 5% of the total cell proteins on SDS-polyacrylamide gel electrophoresis. Since the polyhedrin is unessential for the infection and replication of NPV, conventional baculovirus expression systems utilize the polyhedrin promoter to control expression. Such an expression system consists of a shuttle vector that can be amplified in bacteria and subsequently transfected in vitro into Sf9 cells. The vector is formed of bacterial plasmid elements, e.g., plasmid origin of replication, ampicillinase drug resistance gene, and AcNPV polyhedrin gene and its promoter. After a foreign gene is inserted adjacent to the polyhedrin gene promoter, the gene is expressed under the same conditions that previously induced polyhedrin expression.

pAc360 is another expression vector using the AcNPV polyhedrin gene promoter pAc360 uses the gene encoding β-galactosidase and upon transfection in Sf9 cells, directs production of β-galactosidase enzyme activity.

While the polyhedrin gene of AcNPV and Bombyx mori NPV (BmNPV) contain a high degree of homology, AcNPV cannot be utilized for protein expression in B. mori or its cells in culture. As described above, the AcNPV baculovirus expression system has been used widely to express foreign genes, but expression utilizing AcNPV is limited to the Sf9 cell line due to lack of cross-species infectivity. The present invention enables expression of foreign genes in B. mori silkworm and also increases the stability of the expression products in the hemolymph of silkworm.

NPV virus is obtained from a suitable insect host, preferably Bombyx mori (silkworm), at any appropriate stage of development. Such stages occur both during larval growth and in the adult life of the insect. However, preferably the NPV virus is obtained at a single stage of larval growth, most preferably at the fifth instar stage of silkworm larval development.

Virus is isolated from the insect tissue using conventional methods. Such methods include homogenizing insect tissue, or more preferably, breaking open the larval shell to gain access to the hemolymph. This step yields a preparation of viral inclusion bodies which are further purified, preferably by alternating washing in buffered saline with centrifugation. The virus is extracted from inclusion bodies dissolved in alkaline conditions (about pH 10.8) in the presence of proteinase K at 37° C for 2 hours.

The virus can be purified thereafter by ultracentrifugation in density gradients and still further followed by the extraction with phenol, and dialysis for 12-15 hours.

A suitable bacterial plasmid for this invention contains elements of the conventional baculovirus expression vector, namely, the AcNPV polyhedrin gene and promoter. A recombinant gene may be inserted adjacent to the polyhedrin promoter in such a plasmid. One plasmid which may be used to obtain a recombinant BmNPV expression vector which is pAc360-β-gal which contains the β-galactosidase gene was amplified in bacteria such as preferably E. coli JM103 grown in the presence of ampicillin. Plasmid DNA is then extracted from the cells by phenol and purified such as by column chromatography over Sepharose 2B.

A recombinant expression vector able to produce recombinant proteins in silkworm is obtained by cotransfection of virus and plasmid into cultured cells preferably BmNPV DNA and plasmid DNA containing the AcNPV polyhedrin gene, Bm-N cells. To this end, isolated viral and plasmid DNA are combined in solution. A solute, such as calcium chloride or dextran sulfate, is added to the DNA mixture solution to enhance transfection of DNA into the cells. The DNA admixture is combined with cells in tissue culture which are capable of being infected by the selected NPV. Such transfected cells are capable hosts wherein entry of the virus genome into the cell results in productive infection, i.e., multiplication of NPV.

Cells producing recombinant virus are screened initially for expression of the selected recombinant gene, e.g., an enzyme activity such as β-galactosidase. Plaque results when lysogenic infection occurs in such cells. Virus is isolated from all such plaques.

Next, tissue culture cells are directly infected with virus isolated from plaques. As described previously, cells are again selected by their expression of the recombinant gene product. The procedure is then repeated until pure recombinant virus was obtained.

At this stage suitable recombinant virus include the abilities both to infect the appropriate insect cell in culture and to direct high expression of the selected recombinant gene product. The level of expression can be evaluated by measuring levels of enzyme activity normalized per cell. Expression levels can also be evaluated by measuring the amount of expressed protein as detected by SDS-PAGE analysis of total cellular protein. Satisfactory levels of expression of the recombinant gene product are generally equivalent to at least about 30% by weight of the total cellular protein.

Recombinant NPV virus are also screened by the ability to infect the insect in vivo at a stage in the life of the insect, preferably, the fifth instar larvae of silkworm. The level of the expression efficiency should generally be higher, preferably at least 10 fold higher, most preferably 100-fold higher than the insect cells in culture, e.g. expression in Bm-N cells.

As an additional screen for selecting optimal virus for use as an expression vector in an insect host, induction of anti-protease activity in the insect organism is evaluated, since such activity enhances the lifetime of the recombinant gene products.

Illustrations of various preferred embodiments of the present invention are set forth in the following Examples.

### Example 1

Bombyx mori NPV (BmNPV) was collected from fifth instar larvae of NPV-infected silkworms. Inclusion bodies containing BmNPV were pelleted and washed with 0.25 M NaCl. The virus was dissolved by incubation of inclusion bodies in buffer containing 0.1 M Na₂CO₃, pH 10.8, 0.1 N NaCl, 0.1 % SDS, 1 mg/ml protease K, at 37° C for 2 hours. BmNPV DNA was extracted with water-saturated phenol, and then with chloroform. After overnight dialysis, isolated BmNPV DNA retains the ability to direct virus production upon transfection into Bm-N cell.

Plasmid pAc360-β-gal contains the β-gal gene inserted adjacent to and under the control of the AcNPV polyhedrin promoter. This plasmid was amplified in E. coli JM103 grown in the presence of ampicillin. Plasmid DNA was extracted from the cells by phenol and purified by column chromatography over Sepharose 2B.

Plasmid pAc360-β-gal and BmNPV DNA were dissolved in transfection buffer consisting of 20 mM HEPES, pH 7.05, 0.7 mM Na₂HPO₂, 37 mM NaCl, 6 mM glucose, 5 mM KCL, 115 ug/ml bovine thymus DNA and mixed with 2.5 M CaCl₂, incubated at room temperature for 30 minutes. The resulting fine precipitate was added to a plate of, monolayer cell of Bm-N and incubated at 28° C for 7-8 hours. The medium was replaced and the cells were incubated for an additional 3 to 4 days. Subsequently, the transfected cells were diluted with fresh medium and seeded in a 2 ml culture plate containing a monolayer of fresh Bm-N cells. Blue plaques were formed after incubating for 2 hours at 28° C, removing the medium, overlying with 1.5 % Sea plaque Agarose made up in BML/TC-10 medium containing 200 ug/ml X-gal and further incubating in the presence of X-gal-substrate for 4-6 days. The blue plaques without virus occlusion bodies were picked up and used to infect a fresh lawn of βm-N cells.

This procedure was repeated until pure recombinant virus was obtained.

### Example 2

Following transfection, β-galactosidase was expressed in the Bm-N cell as a 110 kDa protein on SDS-PAGE. Expression of β-galactosidase was nearly 30 percent of the total cell proteins. Recombinant NPV virus directed expression of β-galactosidase gene in fifth instar larvae of silkworm. The level of the expression efficiency was 100-fold greater than Bm-N cells.

### Example 3

Beta-galactosidase activity was shown to be produced by cells containing hybrid virus DNA. Bm-N cells were collected and washed with 0.9 % NaCl, mixed with Laemmli sample buffer (60 mM Tris-HCl, pH 6.8, 2% SDS, 5% 2-mercaptoethanol, 10% glycerol, 0.002 % bromophenol blue) and heated in boiling water. The samples underwent electrophoresis in 10 % SDS-PAGE. After electrophoresis the gel was stained by Coommassle blue. The electrophotograph was analyzed by densitometry at 600 um.

Sample (culture medium, cells or silkworm hemolymph) was added to 1.6 ml of buffer consisting of 6 mM Na₂HPO₄, 4 mM NaH₂HPO₄, 10 mM KCl, 1 mM MgSO₄, 50 mM 2-mercaptoethanol, pH 7.0, prewarmed at 28°C before use), mixed with 0.4 ml of 4 mg/ml ortho-nitro-phenol-galactoside (prewarmed at 28°C) and incubated at 28°C for 2 minutes, 1 ml of 1 M Na₂CO₃ was added to stop the reaction. The optical density was determined at 420 um. 1 p mole of ortho-nitro-phenol formed from the enzymatic reaction system in one minute was referred to as 1 unit.

### Example 4

5 ul recombinant virus Inoculum (4x10′ PFU/ml) was injected into the individual larvae of silkworm in the early of the fifth instar and collected the hemolymph at different days after infection. The body fluid was centrifuged at 15,000 rpm for 10 minutes and the supernatant was assayed for the activity of β-galactosidase. The level of expression was 100-fold greater than that in the BmN cell.

### Example 5

Normally, the hemolymph of fifth instar silkworm larvae lack protease activity. During the replication of NPV virus, protease inhibitor activity was that increased compared with normal controls (see Table 1). As is obtained with wild-type NPV, the proteinase inhibitor activity specifically inhibits chymotrypsin.

| Synthesis of the proteinase inhibitors in the hemolymph of silkworm during the replication of NPV | | | |
|---|---|---|---|
| Day | Healthy | NPV (Folds) | Recombinant NPV |
| 1 | 1 | 1 | 1 |
| 2 | 2 | 20 | 20 |
| 3 | 20 | 50 | 60 |
| 4 | 20 | 140 | 140 |
| 5 | 20 | 200 | 200 |

| Table 1 | | | |
|---|---|---|---|
| | | | |

Thus, it is seen that the AcNPV transfer vector containing β-galactosidase gene recombined with the Bombyx mori NPV in Bm-N cell provides a hybrid virus that enables expression of recombinant proteins in the larvae of silkworm in high efficiency.

The hybrid recombination of the polyhedrin gene between AcNPV and BmNPV broadens the use of baculovirus expression system. That is, by the practice of the present invention, each larvae can be served as a microfermenter to produce target polypeptides. Moreover, concurrent with the multiplication of virus in silkworm larvae, proteinase inhibitors accumulate in the hemolymph thereby providing favorable conditions for the stability of the expression products in the hemolymph.

Although several specific embodiments of the invention have been described in detail herein, it is to be understood that the invention is not limited to those embodiments and that various changes and modifications other than those as particularly pointed out alone can be made by one skilled in the art without departing from the scope or spirit of the invention as defined in the following claims.

## Claims

1. An expression vector, comprising:
a foreign promoter; and
Bombyx mori NPV DNA.

2. An expression vector according to Claim 1, wherein said foreign promoter is an Autograph californica nucleopolyhedrosis virus polhyedrin promoter.

3. An expression vector according to Claim 1 or 2, further comprising a structural gene.

4. An expression vector according to Claim 3, wherein said structural gene is β-galactosidase.

5. An expression vector according to Claim 4, wherein said β-galactosidase gene is a pAc360 gene.

6. A transformed silkworm larvae, said silkworm larvae harboring an expression vector, comprising:
a foreign promoter; and
Bombyx mori NPV DNA.

7. An expression vector according to Claim 6, wherein said foreign promoter is an Autograph californica nucleopolyhedrosis virus polhyedrin promoter.

8. An expression vector according to Claim 7, further comprising a structural gene.

9. An expression vector according to Claim 8, wherein said structural gene is β-galactosidase.

10. An expression vector according to Claim 9, wherein said β-galactosidase gene is a pAe360 gene.

11. A process for expressing a gene which encodes a polypeptide, comprising the steps of:
selecting an expression vector comprising a foreign promoter and Bombyx mori NPV DNA;
transforming a silkworm larvae with said expression vector;
culturing said transformed silkworm larvae; and recovering said polypeptide.

12. A process for expressing a gene according to Claim 11, wherein said silkworm larvae is Bombyx mori.

13. A process for expressing a gene according to Claim 11 or 12, wherein said foreign promoter is an Autograph californica nucleopolyhedrosis virus polyhedron promoter.

14. A process for expressing a gene according to Claim 13, wherein said polypeptide is β-galactosidase.

15. A process for producing an expression vector, comprising the steps of:
selecting a heterologous DNA sequence of interest;
inserting said DNA into a vector in operative association therewith to create a shuttle vector;
combining said shuttle vector with infectious viral DNA in solution to create a DNA mixture;
transfecting host cells with said DNA mixture; and
isolating recombinant virus with hybrid promoter from plaques formed in said transfected host cells.

16. A process for producing an expression vector according to Claim 15, wherein said shuttle vector is amplified.

17. A process for producing an expression vector according to Claim 16, wherein said shuttle vector is amplified in bacteria.

18. A process for producing an expression vector according to Claim 15, comprising the further step of purifying said recombinant virus.

19. A process for producing an expression vector according to Claim 18, wherein said recombinant virus is further purified by infecting additional host cells.

20. The recombinant virus product of Claims 15-19.

21. A transfected host cell harboring the recombinant virus product of Claim 20.
